# EUROPEAN PATENT SPECIFICATION

(11) **EP 1 212 100 B1**
(45) Date of publication and mention of the grant of the patent: **06.04.2005**
(21) Application number: 00965903.8
(22) Date of filing: 28.08.2000
(51) Int. Cl.: A61K 47/48, C07K 16/14, C12N 15/13, A61P 17/08

(54) **DELIVERY SYSTEM FOR ANTIDANDRUFF AGENT**
ABGABESYSTEM FÜR EIN MITTEL GEGEN SCHUPPEN
SYSTEME D'ADMINISTRATION D'UN AGENT ANTIPELLICULAIRE

(30) Priority: 16.09.1999 EP 99307356
(43) Date of publication of application: 12.06.2002
(73) Proprietor: UNILEVER PLC, London EC4P 4BQ (GB); UNILEVER N.V., 3013 AL Rotterdam (NL)
(72) Inventor: FRENKEN, Leo, G. J., Unilever Research Vlaardingen, NL-3130 AC Vlaardingen (NL); VAN DER VAART, Jan, M., Unilever Rch. Vlaardingen, NL-3133 AT Vlaardingen (NL)
(74) Representative: Elliott, Peter William
(86) International application number: PCT/EP2000/008380
(87) International publication number: WO 2001/019871

(56) References cited:
- WO-A-94/04678
- M. SCHMIDT ET AL.: "The complete cDNA sequence and expression of the first major allergenic protein of Malassezia furfur, Mal f 1." EUROPEAN JOURNAL OF BIOCHEMISTRY, vol. 246, no. 1, 15 May 1997 (1997-05-15), pages 181-185, XP000876611 Berlin, Germany
- C. CUNNINGHAM ET AL.: "Humoral responses to Malassezia furfur serovars A, B and C in normal individuals of various ages." BRITISH JOURNAL OF DERMATOLOGY, vol. 127, no. 5, November 1992 (1992-11), pages 476-481, XP000876602 London, GB

## Description

### FIELD OF THE INVENTION

The present invention relates to the delivery of active agents to a target site by means of a molecule, preferably an antibody, or fragment thereof, which binds specifically to a micro-organism present at the target site. In particular, the invention relates to the use of antibodies, or fragments thereof, capable of binding specifically to the micro-organism *Malassezia furfur*, to target delivery of active agents to a site at which the micro-organism is present, especially to target the delivery of antimicrobial agents to the scalp for the treatment and/or prevention of dandruff formation.

### BACKGROUND TO THE INVENTION

The use of antibody/antigen binding to target the delivery of an active agent to its intended site of action has previously been described in the literature. As an example, EP-A-0453097 (Unilever) describes the delivery of a variety of therapeutic agents, including cytotoxic agents such as glucose oxidase conjugated to classical (murine) antibodies, or fragments thereof, to a target site which is preferably a microbial species occurring in dental plaque. Suitable target sites which are discussed include *Streptococcus mutans, S*. *sanguis*, *A. viscosus, A*. *naslundii* and *Bacteriodes gingivalis*.

EP-A-0566368 (Unilever) describes cosmetic compositions for topical application to the skin and/or hair comprising particles enclosing a benefit agent, preferably an antimicrobial agent having a molecular weight not greater than 2000, and a means for binding to an organic surface at the target location. Liposomes enclosing as benefit agent the antimicrobial biphenolic agent Triclosan targeted to micro-organisms present at the target location, such as *Staphylococcus epidermis*, *Proteus vulgaris* and *Coryneform hofmanni*, by means of a lectin as targeting molecule are exemplified. The use of monoclonal or polyclonal antibodies as the means for attaching the particles of the composition to the target is proposed but not exemplified. Similarly, micro-organisms of the genera *Malassezia* are included within the general list of those micro-organisms to which the particles may be suitably be bound but again no example is provided.

Immune responses in patients with severe skin disorders caused by *M. furfur* have been studied by Johansson et al, Acta. Derm. Venerol, 71, 11-16 (1991). Classical (murine) antibodies specific for Malf1, a cell surface protein of *M. furfur,* have been isolated and described by Zagari et al, Allergy, 49, 50-56 (1994) and Schmidt et al, FEBS, 246, 181-185 (1997).

Schmidt *et al*, European Journal of Biochemistry, 246, no 1, May 1997, pages 181-185 discloses the complete cDNA sequence encoding a major allergen of *Malassezia furfur*.

Cunningham *et al*, Brit Journal Dermatology, 127, no 5, November 1992, pages 476-481 describes the humoral responses to *Malassezia furfur* serovars A, B and C in individuals and age-related differences in the responses.

The exact role of *Malassezia furfur* in the formation of dandruff is not known, but this organism has been implicated in dandruff formation on many occasions see, for example, Journal of the Society of Cosmetic Chemists, 18, 629-639 (1967), Van der Wijk; Ingham et al, Journal of Medical and Veterinary Mycology, 31, 265-298 (1993); Danby et al, J. American Acad. Dermatology, 29, 1008-1012 (1993); Schmidt, Cutis, 59, 21-24 (1997). Compared to persons without dandruff, significantly increased numbers of *M*. *furfur* were found on the scalp of persons with dandruff (Priestly et al, Brit. Journal Dermatology, 94, 496-471 (1976); Shuster, Brit. Journal Dermatology, 11, 235-242 (1984)).

Furthermore, a correlation was shown between *M. furfur* numbers on the scalp and dandruff severity (Pierard-Franchimont et al, J. Mycologie Medicale, 8, 83-86 (1998)). Their results showed that a reduction of the number of *M. furfur* cells on persons suffering from dandruff resulted in a decrease in the severity of dandruff.

To date, the treatment and/or prevention of dandruff has involved the use of chemical anti-fungal compounds. Compounds such as ketoconazole (see Pierard-Franchimont et al, above), selenium sulfide (see Danby et al, above), ciclopirox olamine, piroctone-olamine, zinc pyrithione and sulfur-containing substances (see Schmidt, above) climbazole and coal tar are used or tested for the treatment of dandruff. There has been no suggestion in the literature that antibody-mediated delivery of antidandruff agents to the scalp would be beneficial. The use of anti-keratin antibodies which bind to damaged hair has been described by Uchiwa, J. Soc. Cosmet. Chem., (1997). He showed that anti-keratin antibodies with two binding sites could increase resistance to stretching and inhibit damage to hair. No mention was made of antibodies binding and delivering compounds to *M. furfur.*

European Journal of Biochemistry, 1997, 246: 181-185 describes a composition comprising a molecule capable of binding specifically to Malassezia furfur. No mention is made of conjugating such a molecule with an active agent.

There remains a continuing need for the development of new and improved methods for delivering active agents to their intended site of action. In particular, there remains a continuing interest in the development of methods which can be applied economically on a scale appropriate for industrial use.

### SUMMARY OF THE INVENTION

In a first aspect, the invention provides a composition for topical application comprising an active agent with an antimicrobial activity conjugated with a molecule, such as an antibody, or fragment thereof, capable of binding specifically to the micro-organism *Malassezia furfur*.

It will be appreciated that although the invention is described hereinafter primarily by reference to antibodies, or fragments thereof, capable of binding specifically to *Malassezia furfur*, it is equally applicable to other molecules capable of functioning as specific binding ligands in the same way. References to antibodies, or fragments thereof, will accordingly be understood to refer also to such other molecules unless the context dictates otherwise.

As used herein, an "active agent" should be broadly understood and refers to an agent which produces an antimicrobial effect at a target location accessible by topical application, preferably by topical application to the body surface.

An 'antibody' refers to an immunoglobulin which may be derived from natural sources or synthetically produced. Unless indicated otherwise, 'antibody' and immunoglobulin are used synonymously throughout the present specification. An 'antibody fragment' is a portion of a whole antibody which retains the ability to exhibit antigen-binding activity.

A 'functional equivalent' of an immunoglobulin variable domain is any homologous protein molecule which has similar binding specificity. A functionally equivalent protein can be characterised by an insertion, deletion or substitution of one or more amino acid residues in the sequence of the immunoglobulin variable domain. Suitably, the amino acid sequence of the functional equivalent has at least 60% similarity, preferably at least 80%, more preferably at least 90% similarity to the amino acid sequence of the immunoglobulin variable domain.

The present invention may be more fully understood with reference to the following description.

### DETAILED DESCRIPTION OF THE INVENTION

The invention relates to the use of molecules, especially antibodies or fragments thereof, which bind specifically with the micro-organism *Malassezia furfur* to target active agents to sites at which the micro-organism is present. In particular, the invention finds application in the treatment and/or prevention of dandruff formation.

Antibody-mediated delivery of active agents to a target site at which M. furfur is present has not previously been described in the literature. A major difficulty associated with the development of effective active systems lies in ensuring delivery of the active agent selectively to the intended site. The antibodies of the present invention can be used to target active agents selectively to sites where *M. furfur* is present, the combination of site discrimination with binding leading to a reduction in the amount of active agent required for acceptable efficacy whilst achieving retention of the active agent at the target site.

Advantageously, by selectively targeting the active agent to the intended site of action, less active agent is required to achieve the same efficacy. Compositions which are faster acting, longer lasting, and more effectively retain the active agent at the target site (for example, post-rinsing if a rinse-off formulation is used) may be provided in this way. Further a higher ratio of deposition of an antidandruff active agent on the scalp rather than on hair fibre leads to sensory improvements, as coarseness of the hair that might otherwise result through deposition of insoluble active agents on the hair fibre is reduced.

Preferably, the invention relates to the use of heavy chain variable domains derived from an immunoglobulin naturally devoid of light chains, or functional equivalents thereof, such as are obtainable from lymphoid cells, especially peripheral blood lymphocytes, bone marrow cells or spleen cells derived from Camelids as described in WO 94/04678 (Casterman et al). Such immunoglobulins, hereinafter "heavy chain immunoglobulins" do not rely upon the association of heavy and light chain variable domains for formation of the antigen-binding site (as with classical antibodies) but instead the heavy polypeptide chains alone naturally form the complete antigen binding site.

It will be appreciated that heavy chain variable domains derived from other immunoglobulins modified ('Camelised') to enable them to function as monovalent binding domains in the same way as heavy chain variable domains derived from Camelids may also suitably be used according to the invention.

One advantage of using the antibodies of the invention is that they can readily and conveniently be produced economically on a large scale, for example using a transformed lower eukaryotic host as described in WO 97/25591 (Unilever), which process produces only a low level of impurities, requiring only simple downstream processing procedures for purification.

A further advantage of the antibodies of the invention when used in consumer goods products, such as personal care products, is that they are stable both under the conditions used in the processes for their manufacture and under the intended product application conditions.

Not only is it economically unfeasible to use classical "four-chain" immunoglobulins or fragments thereof (produced, for example using conventional hybridoma technology) in consumer goods products but such antibodies are generally insufficiently stable for commercial application when formulated under product conditions as they tend to denature, resulting in loss of specificity, in the presence of surfactants.

Particular antibodies heavy chain variable domains (referred to hereinafter as VHH fragments) capable of binding specifically to *M. furfur* of use according to the invention comprise the sequences:

Heavy chain variable domains derived from an immunoglobulin naturally devoid of light chains having a determined antigen specificity may conveniently be obtained by screening expression libraries of cloned fragments of genes encoding Camelid immunoglobulins generated using conventional techniques, as described, for example, in WO 94/04678 and Example 1. Suitable methods to enrich for binding domains recognising the micro-organism *M*. *furfur,* thereby limiting the number of clones which have to be screened for the identification of binding fragments are yeast display (WO 94/01567 from Unilever) or phage display.

Heavy chain variable domains according to the invention may be prepared by transforming a host by incorporating a gene encoding the polypeptide as set forth above and expressing said gene in said host.

Suitably the host or hosts may be selected from bacteria, such as Gram-negative bacteria, for example *E. coli*, and Gram-positive bacteria, for example *B. subtilis* and in particular lactic acid bacteria, lower eukaryotes such as yeasts, for example belonging to the genera *Saccharomyces, Kluyveromyces, Hansenula* or *Pichia*, or moulds such as those belonging to the genera *Aspergillus* or *Trichoderma*.

Preferred hosts for use in connection with the present invention are the lower eukaryotic moulds and yeasts.

Techniques for synthesising genes, incorporating them into hosts and expressing genes in hosts are well known in the art and the skilled person would readily be able to put the invention into effect using common general knowledge.

Proteins for use according to the invention may be recovered and purified using conventional techniques such as affinity chromatography, ion exchange chromatography or gel filtration chromatography.

The binding activity of the binding proteins according to the invention may conveniently be measured by standard techniques known in the art such as enzyme-linked immunoadsorbant assay (ELISA), radioimmune assay (RIA) or by using biosensors.

The micro-organism *Malassezia furfur* is a member of the normal cutaneous flora which preferentially colonises the skin of the head/neck/face region. Antibodies of the present invention capable of binding specifically to this micro-organism can be used to target actives to the hair and/or scalp, particularly for the treatment and/or prevention of dandruff formation.

Various compounds are contemplated as active agents with an antimicrobial activity for delivery to the target site in accordance with the invention. Preferably, the active agent is an antimicrobial protein.

One suitable active agent is an antimicrobial enzyme able to generate a cytotoxic product, for example glucose oxidase. Glucose oxidase catalyses the oxidation of glucose to gluconic acid by molecular oxygen, producing hydrogen peroxide in the process which exhibits toxicity to target cells provided it can be brought close to them.

Other antimicrobial enzymes which may be delivered in accordance with the invention include other oxidases, such as lactate oxidase and galactose oxidase; peroxidases including lactoperoxidase, chloroperoxidase, horse radish peroxidase, myeloperoxidase. Cell wall lytic enzymes such as lysozyme, b1, 3-glucanase, b1, 6-glucanase and chitinase may also be delivered. Furthermore, specific inhibitors which inhibit extracellular enzymes produced by *M. furfur,* such as the lipases described by Ran et al, J. Med. and Vet. Mycology, 31, 77-85 (1993) and Plotkin et al, J. Med. Vet. Mycology, 34, 43-48 (1996), or produced by other organisms on the scalp could be delivered. Such enzymes could be inhibited by any type of compound, such as llama single-chain antibody fragments, which bind to the enzyme. Alternatively, llama antibody fragments could be used, which bind to the substrate of a for *M*. *furfur* essential degradation product. Binding of the substrate could prevent degradation by the enzyme. Alternatively, inhibitory substrate analogues could be delivered, which inactivate the enzyme after binding. *Malassezia furfur* or extracellular enzymes could also be attacked by delivery of proteases such as papain, pepsin or trypsin.

The compositions for delivery according to the invention may include a substrate for enzyme action or it may rely on the enzyme substrate being present at the target site.

Alternatively, antimicrobial agents such as antimicrobial peptides, metal salts, sequestering agents, biphenolic compounds such as Triclosan, chlorhexidine, antifungal agents such as ketoconazole, selenium sulfide, ciclopirox ocamine, piroctone-olamine, metal pyrithiones such as zinc pyrithione, sulphur containing substances, climbazole and coal tar may also find application in the method of the invention.

Conjugates between the antibodies of the invention and the active agent for use according to the invention may be formed by conventional chemical reactions to form covalent bonds. Such methods are well known and standard methods are available (Hermanson, Biconjugate Techniques, Academic Press Inc., San Diego, California (1996)). Alternatively, conjugates may be formed using conventional recombinant DNA techniques.

It will be appreciated that the invention also includes within its scope the use of conjugates formed through non-covalent self-assembly systems, for example, bispecific antibody fragments with both anti-*M. furfur* and anti-active agent specificity. Alternatively, the active agent may be covalently conjugated to an antibody, or preferably a fragment thereof, which binds by antibody-antigen binding to the antibody fragment capable of binding specifically to *M*. *furfur*.

The compositions of the invention may be formulated in conventional manner using techniques well known in the art.

The amount active agent/antibody fragment conjugates incorporated into the compositions of the invention may depend on the type of composition and the exact nature of active agent used. A preferred amount of is from 0.0001 to 5% by weight of the total composition, more preferably from 0.001 to 3% by weight of the composition, most preferably between 0.01 and 0.1% by weight of the composition.

The compositions according to the invention are intended for topical application to the skin (such as the scalp) and/or hair. The compositions may be formulated in any suitable form for topical application generally used in the personal care art, such as hair care compositions, scalp tonics and lotions, skin care lotions and creams, shower gels, bath foams, soaps, deodorants or the like.

A particularly preferred topical composition in accordance with the invention is a hair treatment composition such as a shampoo composition in which at least one surfactant provides a deterging benefit. The deterging surfactant is preferably selected from anionic, nonionic, amphoteric and zwitterionic surfactants, and mixtures thereof.

Suitable anionic surfactants include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium ammonium and mono-, di- and triethanolamine salts.

The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

Nonionic surfactants suitable for use in compositions of the invention may include condensation products of aliphatic (C₈-C₁₈) primary or secondary linear or branched chain alcohols or phenols with alkylene oxides, usually ethylene oxide and generally having from 6 to 30 ethylene oxide groups. Other suitable nonionics include mono- or di-alkyl alkanolamides. Example include coco mono- or di-ethanolamide and coco mono-isopropanolamide.

Further nonionic surfactants which can be included in shampoos for the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group. Suitable alkyl polyglycosides for use in the invention are commercially available and include for example those materials identified as: Oramix NS10 ex Seppic; Plantaren 1200 and Plantaren 2000 ex Henkel.

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine and sodium cocamphopropionate.

The surfactants are present in shampoo compositions of the invention in an amount of from 0.1 to 50% by weight of the composition, preferably from 0.5 to 30% by weight.

Preferably a mixture of surfactants are used whereby the amount of non ionic or amphoteric surfactants are present in a higher amount than anionic surfactant in order to optimise the stability of the active agent/antibody fusion construct. Preferably the ratio of nonionic or amphoteric surfactants to anionic surfactants is in the range 1:5 to 5:18, most preferably 3:11.

Hair treatment compositions in accordance with the invention may also take the form of hair conditioning compositions, which may be rinse of or leave-on hair conditioning compositions or so-called 2 in 1 compositions containing shampoo and conditioner. The conditioning compositions preferably comprise one or more cationic surfactants. The use of cationic surfactants is especially preferred, because these ingredients are capable of providing conditioning benefits to hair.

Examples of cationic surfactants include: quaternary ammonium hydroxides, e.g., tetramethylammonium hydroxide, alkyltrimethylammonium hydroxides wherein the alkyl group has from about 8 to 22 carbon atoms, for example octyltrimethylammonium hydroxide, dodecyltrimethy- ammonium hydroxide, hexadecyltrimethylammonium hydroxide, cetyltrimethylammonium hydroxide, octyldimethylbenzylammonium hydroxide, decyldimethyl-benzylammonium hydroxide, stearyldi-methylbenzylammonium hydroxide, didodecyldimethylammonium hydroxide, dioctadecyldimethylammonium hydroxide, tallow trimethylammonium hydroxide, cocotrimethylammonium hydroxide, and the corresponding salts thereof, e.g., chlorides, Cetylpyridinium hydroxide or salts thereof, e.g., chloride , Quaternium -5, Quaternium -31,Quaternium -18, and mixtures thereof.

In hair conditioning compositions according to the invention, the level of cationic surfactant is preferably from 0.01 to 10%, more preferably 0.05 to 5%, most preferably 0.1 to 2% by weight of the composition.

Hair treatment compositions of the invention may also contain one or more conditioning agents, preferably selected from silicones, protein hydrolysates and quaternised protein hydrolysates and other materials which are known in the art as having desirable hair conditioning properties.

Silicones are the most preferred conditioning agents.

Suitable silicones include volatile and non-volatile silicones, such as for example polyalkylsiloxanes, polyalkylaryl siloxanes, siloxane gums and resins, cyclomethicones, aminofunctional silicones, quaternary silicones and mixtures thereof. Silicone oil is a particularly preferred conditioning agent for hair. The silicone may be in the form of a low viscosity oil which may contain a high viscosity oil or gum in solution. Alternatively, the high viscosity material may be in the form of an emulsion in water. The emulsion may be of high viscosity oil or of a solution of gum in a lower viscosity oil. The particle size of the oil phase may be anywhere in the range from 30 nanometres to up to 20 microns average size.

The silicone oil may suitably be a polydimethylsiloxane with an average particle size of less than 20 microns and preferably less than 2 microns. Small particle size enables a more uniform distribution of silicone conditioning agent for the same concentration of silicone in the composition. Advantageously, a silicone with a viscosity in the range 1-20 million cst is used. The silicone can be cross-linked.

Preferred silicones include polydimethylsiloxanes (of CTFA designation dimethicone) and hydroxylated polydimethylsiloxanes (of CTFA designation dimethiconol). Silicones of the above types are widely available commercially, for example as DC-1784 and DCX2-1391, both ex Dow Corning.

Suitable protein hydrolysates include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

In accordance with the invention, the hair treatment composition may also comprises a polymeric water-soluble cationic polymer as a conditioning agent.

The cationic polymer may be present at levels of from 0.01 to 5%, preferably from about 0.05 to 1%, more preferably from about 0.08% to about 0.5% by weight.

Synthetic or naturally derived polymers having a quaternised nitrogen atom are useful. The molecular weight of the polymer will generally be between 5 000 and 10 000 000, typically at least 10 000 and preferably in the range 100 000 to about 2 000 000.

Representative synthetic quaternised polymers include, for example: cationic copolymers of 1-vinyl-2-pyrrolidine and 1-vinyl-3-methyl-imidazolium salt (e.g., Chloride salt) (referred to in the industry by the Cosmetic, Toiletry, and Fragrance Association, "CTFA". as Polyquaternium-16); copolymers of 1-vinyl-2-pyrrolidine and dimethylaminoethyl methacrylate (referred to in the industry by CTFA as Polyquaternium-11); cationic diallyl quaternary ammonium-containing polymers including, for example, dimethyldiallylammonium chloride homopolymer (referred to in the industry (CTFA) as Polyquaternium 6); mineral acid salts of amino-alkyl esters of homo-and co-polymers of unsaturated carboxylic acids having from 3 to 5 carbon atoms, as described in U.S. Patent 4,009,256; and cationic polyacrylamides as described in WO95/22311.

Representative naturally-derived quaternised polymers include quaternised cellulosic compounds and cationic guar gum derivatives, such as guar hydroxypropyltrimonium chloride. Examples are JAGUAR C-13S, JAGUAR C-15, and JAGUAR-C17, commercially available from Meyhall in their JAGUAR (trademark) series.

Suitable cationic polyacrylamides are described in WO 95/22311.

The composition may further comprise from 0.1 to 5 % of a suspending agent. Examples are polyacrylic acids, cross linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearates, alkanolamides of fatty acids having from 16 to 22 carbon atoms and mixtures thereof. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol materials are available from Goodrich and Carbopol is a trade mark. A further suitable suspending agent is dihydrogenated tallow phthalic acid amide (available from Stepan under the trademark Stepan TAB-2).

Suitable cross linked polymers of acrylic acid and acrylate esters are Pemulen TR1 or Pemulen TR2. A suitable heteropolysaccharide gum is xanthan gum, for example that available as Kelzan mu.

Another ingredient that may advantageously be incorporated into hair treatment compositions of the invention is a fatty alcohol material. The use of these materials is especially preferred in conditioning compositions of the invention, in particular conditioning compositions which comprise one or more cationic surfactant materials. The combined use of fatty alcohol materials and cationic surfactants in conditioning compositions is believed to be especially advantageous, because this leads to the formation of a lamellar phase, wherein the cationic surfactant is dispersed.

Preferred fatty alcohols comprise from 8 to 22 carbon atoms, more preferably 16 to 20. Examples of preferred fatty alcohols are cetyl alcohol and stearyl alcohol. The use of these materials is also advantageous in that they contribute to the overall conditioning properties of compositions of the invention.

The level of fatty alcohol materials is conveniently from 0.01 to 10%, preferably from 0.1 to 5% by weight of the composition. The weight ratio of cationic surfactant to fatty alcohol is preferably from 10:1 to 1:10, more preferably from 4:1 to 1:8, most preferably from 1:1 to 1:4.

A further ingredient that may be desirably included in the hair treatment composition is a pearlescent material. Suitable pearlescent materials include ethylene glycol distearate, ethylene glycol monostearate, guanine and titanium dioxide coated micas, bismith oxychloride, and stearic monoethanol amide. The level of pearlescent material present in the composition is generally 0.1% to 5%, preferably from 0.3% to 3% by weight of the composition.

The hair treatment compositions of the invention are preferably aqueous based. The compositions suitably comprise water in amount of from about 20 to about 99% by weight of the total composition.

The compositions of the invention are preferably rinse-off compositions, i.e., suitable for applying to the hair and/or scalp, left thereon for an appropriate period of time and then rinsed off with water. Thus, shampoos and conditioners are a particularly preferred product form for compositions of the invention.

Hair treatment compositions of the present invention can be formulated as styling formulations such as sprays in aerosol or nonaerosol forms, and so can be dispensed from containers which are propellant - charged aerosol containers, or alternatively pump spray containers operated without any propellant. Alternatively, styling gel, wax, cream or mousse product forms may be employed. Furthermore product forms such as hair masks and hair soaps may also be employed.

When the hair styling compositions are to be dispensed from a pressurised aerosol container, an aerosol propellant must be included in the composition. This agent is responsible for expelling the other materials from the container and forming the hair spray or mousse character.

The propellant gas can be any liquefiable gas conventionally used for aerosol containers. Preferably the density of the propellant or mixture thereof is less than the hair styling concentrate so that pure propellant is not emitted from the container. Examples of suitable propellants include dimethyl ether, propane, n-butane and isobutane, used singly or admixed. Other examples of propellants are nitrogen, carbon dioxide and compressed air.

The amount of the propellant gases is governed by normal factors well known in the aerosol art. For hair stylings the level of propellant is generally from about 3 to about 50%, preferably from about 5 to about 45%, optimally about 35 to 45% of the total composition.

In the hair styling formulations, hair styling resinous polymers commonly known in the art may be employed, such as those described in GB A 2331528.

The amount of resin typically ranges from 0.5 to 10%, preferably 1.5 to 6 % by weight of the hair styling composition.

Depending on the type of composition employed, one or more additional ingredients conventionally incorporated into hair treatment formulations may be included in the compositions of the invention. Such additional ingredients additional antimicrobial agents, foam boosters, perfumes, colouring agents, preservatives, thickeners, viscosity modifiers, buffering or pH adjusting agents.

The invention is further illustrated by way of the following non-limitative examples, in which all percentages are by weight based on total weight of the composition, unless otherwise specified.

### EXAMPLES

The following examples are provided by way of illustration only. Techniques used for the manipulation and analysis of nucleic acid materials were performed as described in Sambrook *et al*, Molecular Cloning, Cold Spring Harbor Press, New York, 2nd Ed (1989), unless otherwise stated.

V_{HH} denotes heavy chain variable domain of heavy chain antibodies.

### EXAMPLE 1. Induction of a humoral immune response in llama.

A male llama was immunised with extract of *M*. *furfur* and rec. Malf1. Both were obtained from Arezou Zargari of the Karolinska hospital in Stockholm, Sweden. For these immunisations, the antigens were dissolved in oil emulsion (1:9 V/V, antigen in PBS : Specol (Bokhout et al, Vet. Immunol. Immunopath, 2, 491-500 (1981)) subcutaneously and intramuscularly. Per immunisation site 0.75-1.5 ml water in oil emulsion was injected containing 1 mg *M. furfur* extract or 200 mg rec. Malf1 protein. Immunisations were performed according to the following time schedule: the second immunisation was performed three weeks after the first injection, and the third immunisation two weeks after the second one. The immune response was followed by titration of serum samples in ELISA with rec. Malf1 immobilised on Nunc maxi-sorb plates (coat solution 5 mg/ml diluted in phosphate buffered saline). After incubation with serum, the bound llama antibodies were detected with poly-clonal rabbit-anti-llama antiserum (obtained via immunising rabbits with llama immunoglobulines purified via ProtA and ProtG columns; ID-DLO) and swine-anti-rabbit immunoglobulines (Dako) conjugated to horse radish peroxidase. Finally the peroxidase enzyme-activity was determined with tetramethylbenzidine and ureaperoxide as substrate and, after termination of the reaction by adding H2SO4, the optical density was measured at 450nm.

### EXAMPLE 2. Cloning, selection and screening of llama VHH fragments which bind Malassezia furfur.

### Isolation of VHH fragments against M. furfur.

From an immunised llama a blood sample of about 200ml was taken and an enriched lymphocyte population was obtained via centrifugation on a Ficoll (Pharmacia) discontinuous gradient. From these cells, total RNA was isolated by acid guanidium thiocyanate extraction (e.g. via the method described by Chomczynnski and Sacchi, Analytical Biochem, 162, 156-159, 1987). After first strand cDNA synthesis using MMLV-RT (Gibco-BRL) and random oligonucleotide primers (Pharmacia), DNA fragments encoding VHH fragments and part of the long or short hinge region were amplified by PCR using specific primers: S = C and G, M = A and C, R = A and G, W = A and T,

The DNA-fragments generated by PCR were digested with PstI (coinciding with codon 4 and 5 of the VHH domain, encoding the amino acids L-Q) and BstEII (naturally occurring in FR4 region of the majority of the VHH-genes and coinciding with the amino acid sequence Q-V-T). The digested PCR-products, with a length between 300 and 400bp (encoding the complete VHH domain, but lacking the first and the last three codons), were separated by electrophoresis on agarosegel and, after purification from the gel-slices with the Qiaex-II extraction kit, cloned in the Saccharomyces cerevisiae episomal expression plasmids pUR4547 or pUR4548. Plasmid pUR4547 (deposited under the Budapest Treaty at Centraalbureau Voor Schimmelcultures, Baarn, The Netherlands, Phabagen collection with deposition number CBS100012 on the 18^{th} of August 1997), with an Ori for autonomous replication in *S. cerevisiae*, enables the production via the inducible *Gal7* promotor; secretion is accomplished by fusing the SUC leader sequence (Harmsen et al (1993), Gene, 125, 115-123) to the amino terminus of the V_{HH} encoding sequence. The expression plasmid pUR4548 (deposited under the Budapest Treaty at Centraal bureau Voor schimmelcultures, Baarn, The Netherlands, Phabagen collection with the deposition number CBS100013 on the 18^{th} August 1997) is identical to pUR4547, but contains a DNA sequence encoding the c-myc tail at the c-terminal end of the V_{HH} encoding sequence. Malf1 specific VHH fragments were isolated via screening of culture supernatants from individual clones in ELISA using a rabbit anti-llama VHH poly-clonal antibody (obtained via immunising rabbits with llama immunoglobulines purified via ProtA and ProtG columns; ID-DLO, for VHH fragments cloned in pUR4547) or the mouse anti-myc mono-clonal antibody 9E10 (Evan et al., Mol. Cell. Biol., 5, 3610-3616 (1985), for VHH genes cloned in pUR4548) for detection (described in paragraph 2.2).

In accordance with Rule 28(4)EPC, or similar arrangement from a state not being a contracting state or the EPC, it is hereby requested that a sample of deposits CBS100012 and CBS100013, when requested, will be submitted to an expert only.

As an alternative, the PCR-products can be digested with PstI and BstEII and cloned in the yeast display vector pUR4659 (DNA encoding the 318 C-terminal amino acids of α-agglutinin was amplified in a PCR with pUR5820 [Van der Vaart *et al*., Appl. And Envo Microbiolo, 63, 615-620 (1997) as a template and a 5' primer containing a *Bst*EII restriction site and a 3' primer downstream of the *Hind*III restriction site. This PCR fragment was cloned (*Bst*EII/*Hin*dIII) into the yeast expression vector pUR4548) as gene-fragments encoding the VHH-domain including the hinge region. VHH fragments capable of binding to rec. Malf1 or whole *M. furfur* cells can be isolated with the selection method based on the principle of yeast display (described in paragraph 2.3-4 and 2.5, respectively).

### 2.2 Screening of S. cerevisiae culture supernatants for M. furfur specific VHH domains.

For the production of llama VHH fragments in *S. cerevisiae*, individual transformants were grown overnight in selective minimal medium (comprising 0.7% yeast nitrogen base, 2% glucose, supplemented with the essential amino acids and bases) and subsequently diluted ten times in YPGal medium (comprising 1% yeast extract, 2% bacto pepton and 5% galactose). After 24 and 48 hours of growth, the culture supernatant of the colonies was analysed by ELISA for the presence of VHH fragments, which specifically bind to immobilised rec. Malf1. Bound VHH fragments were detected with rabbit anti-llama VHH poly-clonal antibodies followed by incubation with goat anti-rabbit poly-clonal antibodies conjugated to horse radish peroxidase (Bio-rad), or with mouse monoclonal anti-myc antibody followed by incubation with poly-clonal goat-anti-mouse conjugate with horse radish peroxidase.

### 2.3 Selection of Malf1 recognising VHH domains that are well secreted by S. cerevisiae and have the desired functionality by yeast display method.

VHH antibody domains were displayed on yeast by means of N-terminal fusions onto the cell wall protein a-agglutinin (see patent WO 94/01567). Such a yeast display library with 1x10⁶ clones was constructed in the episomal yeast expression vector pUR4659. Transformed yeast cells were pooled and grown for about 17 hours on selective minimal medium until the OD660 was 2. Subsequently, these cells were inoculated into YPGal for expression, secretion and display of the VHH antibody fragments on the cell surface and grown for appr. 17 hours. Secreted VHH - fragments were removed by washing the cells with PBS, thereby avoiding a disturbing competition for binding to antigen between yeast bound and free VHH domains. The displaying *S*. *cerevisiae* cells were incubated for two hours with *in vitro* biotinylated rMalf1 in PBS followed by an incubation with FITC-labelled streptavidin. Fluorescence of individual yeast cells was analysed by means of a FACS (Fluorescence Activated Cell Sorter) and the cells with give above average fluorescent signals were selected and inoculated in minimal medium for a second round selection. In this second round, cells with the highest fluorescent signals were selected and propagated as single colonies for analysis or grown for further rounds of selections. Individual *S*. *cerevisiae* clones were grown on YPgal for 48 hours in wells of microtiter plates. During the second part of this incubation, fusion protein consisting of a VHH fragment and a-agglutinin is secreted in the growth-medium. Culture supernatants containing these chimeric molecules were tested in ELISA for binding to rec.Malf1 using the myc-TAG for detection as described above. For further analysis of selected clones, VHH-encoding DNA fragments from these yeast display vectors were recloned in the episomal yeast secretion vector pUR4548 and analysed as described in paragraph 2.2.

### 2.4 Selection of Malf1 recognising VHH domains via yeast display in consumer products under application conditions.

VHH antibody domains were displayed on yeast by means of N-terminal fusions onto the cell wall protein alpha-agglutinin as described in paragraph 2.3. The displaying *S. cerevisiae* cells were incubated for one hour with in vitro biotinylated rec.Malf1 under application conditions relevant to products. In this example we have dissolved the yeast cells in shampoos, resp 10% Andrelon^{R} and in 2% Organics^{R} followed by the addition of the biotinylated rec.Malf1. After a one hour incubation under application conditions, unbound rec.Malf1 and detergents were discarded by centrifugation. Subsequently, the cells were incubated with FITC-labelled streptavidin and analysed as described above (paragraph 2.3).

### 2.5 Selection of M. furfur recognising VHH domains via yeast display.

VHH antibody domains were displayed on yeast as described in paragraph 2.3. This collection of antibody displaying yeast cells was mixed with biotinylated *M*. *furfur* cells for 2 hours. Subsequently, strepavidin labelled magnetic beads were included in this mixture after which they were allowed to bind to the biotinylated *M. furfur* for appr. 1 hour. Yeast cells displaying an antibody fragment binding to *M*. *furfur* were separated from the unbound displaying yeast cells and washed by their ability to become bound to a magnet. After several washes the remaining displaying yeast cells were plated out on selective minimal medium for their recovery. Subsequently, these selected yeast cells can be grown for a second selection round as described above or single colonies can be grown for analysis of single antibody fragments.

### 2.6 Immune-fluorescent microscopy on M. furfur cells with selected antibody fragments.

To determine whether a selected antibody fragment is specific for a cell surface exposed antigen of *M. furfur,* immuno fluorescent microscopy was performed. *M. furfur* cells were cultured on Dixon's-medium (3,6% w/v malt extract, 0,6% w/v pepton, 2% w/v ox-bile decicated 1,0% v/v tween 40, 0,2% v/v glycerol, 0,2% v/v oleic acid, 0.005% w/v chloramphenicol, 0,04% w/v cycloheximide) at 37 °C for 4 days. Subsequently, cells were incubated with antibody fragments specifically recognising rec. Malf1 and/or the *M. furfur* extract in the performed ELISA tests. After this incubation cells were washed 2 times with PBS and further incubated with mouse monoclonal anti-myc antibody followed by an incubation with FITC-labelled poly-clonal goat-anti-mouse antiserum. Subsequently, cells were analysed by immunofluorescent microscopy.

### Sequences of M. furfur recognising VHH fragments.

In the way described in the paragraphs above a number of anti-Malf1 fragments were obtained. The sequence of three of these are presented below:

Clone selected in PBS: VHH#1 (P_A7); clone selected in 10% Andrelon: VHH#2 (AN_D12) and Clone selected in 2% Organics: VHH#3 (OR_F11) (all cloned in E. coli production plasmid pUR3827 or in *S*. *cerevisiae* episomal expression plasmid pUR4548):

### EXAMPLE 3: Creation of antibody fragment active agent fusion constructs

### 3.1 Creation of chimeric molecules consisting of a VHH and glucose oxidase for the selective killing of M. furfur.

For the selective killing of *M*. *furfur,* a chimeric protein was created consisting of an anti *M*. *furfur* antibody fragment and the redox enzyme glucose oxidase. By coupling the gene encoding an anti *M*. *furfur* VHH to the gene encoding glucose oxidase (Gene sequence from Dactylium dendroides, EMBL Ac. No. M86819), it becomes possible to target this redox enzyme to the dandruff causing yeast. Due to this targeting effect much lower quantities of enzyme will be necessary for the killing of *M. furfur* and, furthermore, less damage will be caused to the other micro-organisms present on the scalp.

To create a fusion construct containing glucose oxidase (N-terminal) and the antibody fragment VHH#2 (AN_D12, C-terminal), an XhoI restriction site should be introduced at the N-terminal end of the VHH#2. This can be achieved in two sequential splicing by overlap PCR reactions (Horton *et al;* Methods in Enzymology, 217, 270-279 (1993)). In the first reaction the DNA sequence for the complete glucose oxidase gene is fused upstream of the GAL7 promoter. The left-hand GAL7 promoter region is amplified by PCR with pUR4548 (see above) as a template and MAL09 (5' GGGAGAGGATCCAAAAAGCG 3', containing *Bam*HI) and MAL10 (5' CAAGGAGAGTCCTCATGATTTTGTTTTGTTTGTTTG 3') as primers. The right-hand fragment containing the complete GOX gene was amplified by using chromosomal DNA of Dactylium dendroides as a template and MAL11 (5' ATGAGGACTCTCCTTGTGAG 3') and MAL12 (5' GACTCCTGCAGCTGGACCTGCTGCATGGACGCGTAATC 3') as primers. Subsequently, both fragments were added as templates in a third PCR and spliced by adding the MAL09 and MAL12 primers. The resulting PCR fragment will serve now as a left-hand fragment in a second splicing by overlap PCR. The right-hand fragment will contain the DNA sequence of VHH#2 and is created in a PCR with pUR4548 containing VHH#2 (see above) as a template and MAL13 (5' CAGGTCCAGCTGCAGGAGTC 3') and MAL14 (5' CGCAAGCTTTCATGAGGTGACGGTGACCTGGG 3', containing HindIII) as primers. These two PCR products can be spliced in a final PCR with both PCR fragments as template and MAL09 and MAL14 as primers. Finally, this DNA fragment is digested with *Bam*HI and *Hin*dII and cloned into pUR4548 (see above). The resulting plasmid is a yeast episomal expression plasmid containing the GAL7 promoter, the DNA sequence encoding glucose oxidase, including the signal sequence (EMBL Ac. No. M86819), and the DNA sequence encoding the antibody fragment VHH#2 (AN_D12). This plasmid can be introduced in Saccharomyces cerevisiae for expression of the glucose-oxidase- VHH#2 fusion construct.

### 3.2 Creation of chimeric molecules consisting of a VHH and a glucanase for the selective killing of M. furfur.

For the selective killing of *M*. *furfur,* a chimeric protein was created consisting of an anti *M. furfur* antibody fragment and the glucanase enzyme b1,3-glucanase and/or b1,6-glucanase. By coupling the gene encoding an anti *M. furfur* VHH to the gene encoding glucanase (gene sequence for b1,6-glucanase from *Pichia harzianum*, EMBL Ac. No. X79196), it becomes possible to target this enzyme to the dandruff causing yeast. The enzymes are capable of degrading the cell wall of the yeast which will result in lysis. The b1,6-glucanase enzyme and VHH antibody fragments have been produced in various lower eukaryotes, including Pichia *pastoris*.

The b1,6-glucanase encoding DNA sequence can be fused to the N-or C-terminal end of the heavy chain antibody fragment.

### Fusion to the N-terminal end of VHH#2 (AN_D12):

This fusion construct can be created by means of a splicing by overlap extension PCR reaction. The left-hand fragment of the fusion fragment, containing the complete b1,6-glucanase encoding DNA sequence (including pro-enzyme peptide), can be amplified from plasmid pUR3421 (the DNA fragment encoding the b1,6-glucanase enzyme was isolated by PCR with chromosomal DNA of *Trichoderma* harzianum as template and JC-THGL1 [5' CGGCGGATCCAAACGATGAAGTACTCCATCGTTGC 3'] and JC-THGL2 [5' CGGCGAATTCTTACCTGAATCCAGCGCAGAC 3'] as primers. This fragment was cloned (*Bam*HI/*Eco*RI) into pPIC9k from invitrogen, The Netherlands) with the 5' primer MAL01 (5'-CTGCTGATAGCCTAACGTTC-3') and the 3' primer MAL02 (5'-GGACTCCTGCAGCTGCACCTGCCTGAATCCAGCGCAGACATCC-3'). The right-hand fragment of the fusion fragment, containing the VHH#2 (AN_D12) encoding DNA sequence, can be amplified from plasmid pUR4548 (CBS100013), which contains the VHH#2 antibody fragment (see example 2), with the 5' primer MAL03 (5'-GGATGTCTGCGCTGGATTCAGGCAGGTGCAGCTGCAGGAGTCC-3') and the 3' primer MAL04 (5'-CGCGAATTCTTATGAGGAGACGGTGACCTGGG-3'). These two PCR products can be used in a second PCR as templates with primers MAL01 and MAL04. The complete fusion construct can be cloned into pUR3421 (BamHI/EcoRI, see above) for expression in *Pichia pastoris*.

### Fusion to the C-terminal end of VHH#2 (AN_D12):

This fusion construct can also be created by means of a splicing by overlap extension PCR reaction). The left-hand fragment of the fusion fragment, containing the VHH#2 (AN_D12) encoding DNA sequence including the invertase signal sequence, can be amplified from plasmid p4548 (CBS100013), which contains the VHH#2 antibody fragment (see example 2), with the 5' primer MAL05 (5'-CGCGGATCCATGCTTTTGCAAGCCTTCC-3') and the 3' primer MAL06 (5'-CCAGAAGCAAGGGCAGGCTCAAATGAGGAGACGGTGACCTGGG-3'). The right-hand fragment of the fusion fragment, containing the b1,6-glucanase encoding DNA sequence devoid of signal sequence and pro-enzyme peptide, can be amplified from plasmid pUR3421 (see above) with the 5' primer MAL07 (5'-CCCAGGTCACCGTCTCCTCATTTGAGCCTGCCCTTGCTTCTGG-3') and the 3' primer MAL08 (5'-GGAACAGTCATGTCTAAGGCG-3'). These two PCR products can be used in a second PCR as templates with primers MAL05 and MAL08. The complete fusion construct can be cloned into pUR3421 (*Bam*HI/*Eco*RI) for expression in *Pichia pastoris*.

### EXAMPLES 4 and 5

Hair shampoo compositions incorporating the antibody fragment/active agent fusion constructs according to example 3 above were formulated as outlined below.

| **INGREDIENT** | **% BY WEIGHT** | **% BY WEIGHT** |
|---|---|---|
| | **EXAMPLE 4** | **EXAMPLE 5** |
| Sodium Laureth Sulfate (70% active) | 15.714 | 15.714 |
| | | |
| Cocamidopropyl Betaine (30% active) | 10 | 10 |
| | | |
| Jaguar C135 | 0.1000 | 0.1000 |
| | | |
| Hydroxypropyltrimonium Chloride DMDM Hydantoin | 0.3000 | 0.3000 |
| | | |
| Tocopheryl Acetate | 0.010 | 0.010 |
| | | |
| Carbopol 980 | 0.6000 | 0.6000 |
| | | |
| Sodium Hydroxide | 0.220 | 0.220 |
| | | |
| Antibody fragment/active agent fusion construct of example 3.1 | 0.100 | 0.000 |
| | | |
| Antibody fragment/active Agent fusion construct of example 3.2 | 0.000 | 0.100 |
| | | |
| Zinc Sulfate | 0.100 | 0.100 |
| | | |
| Sodium Chloride | 1.2000 | 1.2000 |
| | | |
| Preservative, colour, perfume | - q.s - | - q.s - |
| | | |
| Water | - to 100% - | - to 100% - |

These shampoos gave excellent anti-dandruff performance on the hair.

## Claims

1. A composition for topical application comprising an active agent with an antimicrobial activity conjugated with a molecule capable of binding specifically to *Malassezia furfur*.

2. A composition according to claim 1, wherein the active agent is conjugated with an antibody, or fragment thereof, capable of binding specifically to *Malassezia furfur*.

3. A composition according to claim 2, wherein the antibody, or fragment thereof, comprises a heavy chain variable domain derived from an immunoglobulin naturally devoid of light chains, or a functional equivalent thereof.

4. A composition according to any one of claims 1 to 3, wherein the active agent comprises an oxidative enzyme.

5. A method for preparing a composition according to any of claims 1 to 4, comprising conjugating an active agent with a molecule capable of binding specifically to *Malassezia furfur*.

## Patentansprüche

1. Zusammensetzung zur topischen Anwendung, umfassend einen Wirkstoff mit antimikrobieller Aktivität, der mit einem Molekül konjugiert ist, das zur spezifischen Bindung an *Malassezia furfur* befähigt ist.

2. Zusammensetzung nach Anspruch 1, wobei der Wirkstoff mit einem Antikörper oder einem Fragment davon konjugiert ist, der bzw. das zur spezifischen Bindung an *Malassezia furfur* befähigt ist.

3. Zusammensetzung nach Anspruch 2, wobei der Antikörper oder das Fragment davon eine variable Domäne der schweren Kette oder ein funktionelles Äquivalent davon umfasst, die bzw. das von einem Immunglobulin abgeleitet ist, das natürlicherweise keine leichten Ketten enthält.

4. Zusammensetzung nach einem der Ansprüche 1 bis 3, wobei der Wirkstoff ein oxidatives Enzym umfasst.

5. Verfahren zur Herstellung einer Zusammensetzung nach einem der Ansprüche 1-4, umfassend das Konjugieren eines Wirkstoffs mit einem Molekül, das zur spezifischen Bindung an *Malassezia furfur* befähigt ist.

## Revendications

1. Composition pour application topique comprenant un agent actif ayant une activité antimicrobienne, conjugué à une molécule capable de se lier spécifiquement à *Malassezia furfur*.

2. Composition selon la revendication 1, dans laquelle l'agent actif est conjugué à un anticorps, ou à un fragment de celui-ci, capable de se lier spécifiquement à *Malassezia furfur*.

3. Composition selon la revendication 2, dans laquelle l'anticorps, ou le fragment de celui-ci, comprend un domaine variable à chaîne lourde dérivé d'une immunoglobuline naturellement dépourvue de chaînes légères, ou un équivalent fonctionnel de celui-ci.

4. Composition selon l'une quelconque des revendications 1 à 3, dans laquelle l'agent actif comprend une enzyme oxydante.

5. Méthode pour la préparation d'une composition selon l'une quelconque des revendications 1 à 4, comprenant la conjugaison d'un agent actif avec une molécule capable de se lier spécifiquement à *Malassezia furfur*.


### SEQUENCE LISTING

<110> UNILEVER PLC
<120> DELIVERY SYTEM FOR ANTI-DANDRUFF AGENT
<130> J3501
<140> 99307356.8
   <141> 1999-09-16
<160> 22
<170> PatentIn Ver. 2.1
<210> 1
   <211> 119
   <212> PRT
   <213> LLAMA
<400> 1
   Gln Val Gln Leu Gln Glu Ser Gly Gly Gly Leu Val Gln Ala Gly Gly
    1               5                  10                  15
   Ser Leu Arg Leu Ser Cys Gln Ala Ser Gly Asn Ile Phe Arg Ile Asn
               20                  25                  30
   Asp Met Gly Trp Tyr Arg Gln Ala Pro Gly Thr Gln Arg Glu Leu Val
           35                  40                  45
   Ala Ala Ile Thr Thr Gly Gly Ser Thr Lys Tyr Ala Asp Ser Val Lys
       50                  55                  60
   Gly Arg Phe Thr Ile Ser Arg Asp Asn Ala Lys Asn Thr Val Tyr Leu
    65                 70                  75                  80
   Gln Met Asn Ser Leu Lys Pro Glu Asp Thr Ala Val Tyr Tyr Cys Ala
                   85                  90                  95
   Ala Glu Asp Arg His Arg Ile Gly Thr Val Gly Tyr Trp Gly Gln Gly
               100                 105                 110
   Thr Gln Val Thr Val Ser Ser
           115
<210> 2
   <211> 119
   <212> PRT
   <213> LLAMA
<400> 2
   Gln Val Gln Leu Gln Glu Ser Gly Gly Gly Leu Val Gln Ala Gly Gly
    1               5                  10                  15
   Ser Leu Arg Leu Ser Cys Gln Ala Ser Gly Asn Ile Phe Arg Ile Asn
               20                  25                  30
   Asp Met Gly Trp Tyr Arg Gln Ala Pro Gly Thr Arg Arg Glu Leu Val
           35                  40                  45
   Ala Ala Ile Thr Ser Gly Gly Ser Thr Lys Tyr Ala Asp Ser Val Lys
       50                  55                  60
   Gly Arg Phe Thr Ile Ser Arg Asp Asn Ala Lys Asn Thr Val Tyr Leu
    65                 70                  75                  80
   Gln Met Asn Ser Leu Lys Pro Glu Asp Thr Ala Val Tyr Tyr Cys Ala
                   85                  90                  95
   Ala Glu Asp Arg His Arg Val Gly Thr Val Gly Tyr Trp Gly Gln Gly
               100                 105                 110
   Thr Gln Val Thr Val Ser Ser
           115
<210> 3
   <211> 119
   <212> PRT
   <213> LLAMA
<400> 3
   Gln Val Gln Leu Gln Glu Ser Gly Gly Gly Leu Val Gln Ala Gly Gly
   1               5                   10                  15
   Ser Leu Arg Leu Ser Cys Gln Ala Ser Gly Asn Ile Phe Arg Ile Asn
               20                  25                  30
   Asp Met Gly Trp Tyr Arg Gln Ala Pro Gly Thr Arg Arg Glu Leu Val
           35                  40                  45
   Ala Ala Ile Thr Ser Glu Gly Ser Thr Lys Tyr Ala Asp Ser Val Lys
       50                  55                  60
   Gly Arg Phe Thr Ile Ser Arg Asp Asn Ala Glu Asn Thr Val Tyr Leu
   65                  70                  75                  80
   Gln Met Asn Ser Leu Lys Pro Glu Asp Thr Ala Val Tyr Tyr Cys Ala
                   85                  90                  95
   Ala Glu Asp Arg His Arg Ile Gly Thr Val Gly Tyr Trp Gly Gln Gly
               100                 105                 110
   Thr Gln Val Thr Val Ser Ser
           115
<210> 4
   <211> 22
   <212> DNA
   <213> Artificial Sequence
<220>
   <223> Description of Artificial Sequence:PRIMER
<400> 4
   aggtsmarct gcagsagtcw gg                                          22
<210> 5
   <211> 53
   <212> DNA
   <213> Artificial Sequence
<220>
   <223> Description of Artificial Sequence:PRIMER
<400> 5
   aacagttaag cttccgcttg cggccgcgga gctggggtct tcgctgtggt gcg        53
<210> 6
   <211> 53
   <212> DNA
   <213> Artificial Sequence
<220>
   <223> Description of Artificial Sequence:PRIMER
<400> 6
   aacagttaag cttccgcttg cggccgctgg ttgtggtttt ggtgtcttgg gtt 53
<210> 7
   <211> 20
   <212> DNA
   <213> Artificial Sequence
<220>
   <223> Description of Artificial Sequence:PRIMER
<400> 7
   gggagaggat ccaaaaagcg 20
<210> 8
   <211> 36
   <212> DNA
   <213> Artificial Sequence
<220>
   <223> Description of Artificial Sequence:PRIMER
<400> 8
   caaggagagt cctcatgatt ttgttttgtt tgtttg 36
<210> 9
   <211> 20
   <212> DNA
   <213> Artificial Sequence
<220>
   <223> Description of Artificial Sequence:PRIMER
<400> 9
   atgaggactc tccttgtgag 20
<210> 10
   <211> 38
   <212> DNA
   <213> Artificial Sequence
<220>
   <223> Description of Artificial Sequence:PRIMER
<400> 10
   gactcctgca gctggacctg ctgcatggac gcgtaatc 38
<210> 11
   <211> 20
   <212> DNA
   <213> Artificial Sequence
<220>
   <223> Description of Artificial Sequence:PRIMER
<400> 11
   caggtccagc tgcaggagtc 20
<210> 12
   <211> 32
   <212> DNA
   <213> Artificial Sequence
<220>
   <223> Description of Artificial Sequence:PRIMER
<400> 12
   cgcaagcttt catgaggtga cggtgacctg gg 32
<210> 13
   <211> 35
   <212> DNA
   <213> Artificial Sequence
<220>
   <223> Description of Artificial Sequence:PRIMER
<400> 13
   cggcggatcc aaacgatgaa gtactccatc gttgc 35
<210> 14
   <211> 31
   <212> DNA
   <213> Artificial Sequence
<220>
   <223> Description of Artificial Sequence:PRIMER
<400> 14
   cggcgaattc ttacctgaat ccagcgcaga c 31
<210> 15
   <211> 20
   <212> DNA
   <213> Artificial Sequence
<220>
   <223> Description of Artificial Sequence:PRIMER
<400> 15
   ctgctgatag cctaacgttc 20
<210> 16
   <211> 43
   <212> DNA
   <213> Artificial Sequence
<220>
   <223> Description of Artificial Sequence:PRIMER
<400> 16
   ggactcctgc agctgcacct gcctgaatcc agcgcagaca tcc 43
<210> 17
   <211> 43
   <212> DNA
   <213> Artificial Sequence
<220>
   <223> Description of Artificial Sequence:PRIMER
<400> 17
   ggatgtctgc gctggattca ggcaggtgca gctgcaggag tcc 43
<210> 18
   <211> 32
   <212> DNA
   <213> Artificial Sequence
<220>
   <223> Description of Artificial Sequence:PRIMER
<400> 18
   cgcgaattct tatgaggaga cggtgacctg gg 32
<210> 19
   <211> 28
   <212> DNA
   <213> Artificial Sequence
<220>
   <223> Description of Artificial Sequence:PRIMER
<400> 19
   cgcggatcca tgcttttgca agccttcc 28
<210> 20
   <211> 43
   <212> DNA
   <213> Artificial Sequence
<220>
   <223> Description of Artificial Sequence:PRIMER
<400> 20
   ccagaagcaa gggcaggctc aaatgaggag acggtgacct ggg 43
<210> 21
   <211> 43
   <212> DNA
   <213> Artificial Sequence
<220>
   <223> Description of Artificial Sequence:PRIMER
<400> 21
   cccaggtcac cgtctcctca tttgagcctg cccttgcttc tgg 43
<210> 22
   <211> 21
   <212> DNA
   <213> Artificial Sequence
<220>
   <223> Description of Artificial Sequence:PRIMER
<400> 22
   ggaacagtca tgtctaaggc g 21